# EUROPEAN PATENT APPLICATION

(11) **EP 4 631 518 A1**
(43) Date of publication of application: **15.10.2025**
(21) Application number: 24020114.5
(22) Date of filing: 08.04.2024
(51) Int. Cl.: A61K 38/03, A61K 38/07, A61P 11/00

(54) **COMPOSITIONS FOR TREATING DISEASES OF THE AIRWAYS**

(71) Applicant: The Boots Company plc, Nottingham NG90 1BS (GB)
(72) Inventor: BELL, Michael david, Alfreton, derbyshire, DE 55 6EL (GB); BRADLEY, Eleanor Jane, Beeston, Notttingham, NG9 2ES (GB); SHERATT, Michael John, High Peak, SK23 7SB (GB)
(74) Representative: Mukhtar, Amira

(57) **Abstract**

A composition for use in the treatment of disease of the airway in a subject and methods for the same, and a composition suitable for inhalation and kits comprising said composition in combination with an inhaler or nebulizer, the compositions comprising tetrapeptides, peptides, or combinations thereof.

## Description

### Technical Field

The present invention relates to compositions capable of treating diseases of the airways in a subject, uses and methods of such compositions, and a kit comprising such compositions and an inhaler or nebuliser.

### Background to the invention

Disease of the airways include asthma, acute respiratory distress syndrome (ARDS), asbestosis, bronchiectasis, bronchitis, catarrh, chronic obstructive pulmonary disease (COPD), cough, croup, cystic fibrosis, idiopathic pulmonary fibrosis (IPF), obstructive sleep apnoea, pleurisy, pneumonia, pulmonary hypertension, or pulmonary fibrosis.

Current treatment methods for such conditions generally focus on immediately improving gaseous exchange through airway opening - e.g. bronchodilation via adrenergic receptor activation (Salbutamol) or muscarinic receptor antagonism (Ipratropium), reducing mucus build up in the lungs with mucolytics, and antibiotics in the case of infections or anti-inflammatory substances such as corticosteroids.

These treatment methods help tackle acute respiratory distress and inflammation that does not fully resolve, and in most cases are short acting. Long term use of higher dose steroid inhalers is associated with higher risk of a number of conditions including diabetes and obesity, as well as yeast and/or fungal infections. There are no interventions that currently focus on the repair of damaged matrix to improve respiratory function in the long term. A damaged extracellular matrix (ECM) can make the lungs more susceptible to future infections/disease with scarring also reducing respiratory function. There would therefore be considerable benefits in repairing this damage.

WO2022106054A1 discloses a peptide combination for use in cosmetic beauty applications, and compositions comprising said peptide combinations. This document teaches the benefits of the peptide combinations in relation to enhancing production of dermal ECM proteins (such as collagen, fibrillin, fibronectin and decorin) in aging skin.

### Summary of the invention

The present inventors have identified that specific peptides previously used as cosmetic skin treatments are effective in treating diseases of the airways and promoting repair of damaged airways. The specific peptides utilised in the present invention enhance production of dermal ECM proteins (such as collagen, fibrillin fibronectin and decorin).

Lung damage and disease is characterised by altered ECM composition and quality. Injury and subsequent repair invariably lead to scar tissue formation. Such scar tissue formation can become progressive leading to pulmonary fibrosis in which the ECM becomes stiff due to an im-balance in the production of key matrix proteins such as collagen and elastic fibre proteins such as fibrillin. The regulated remodelling of the ECM, especially in healing following a disease, such as a lung disease, by its resident cells, is a prerequisite for maintenance of tissue homeostasis. In pathological events, qualities of the ECM, such as its composition and topological features, biomechanical properties, or role as a reservoir for secreted mediators, act as instructive cues that affect the behaviour of fibroblasts and other cell types. Once tissue homeostasis is disturbed by repeated injury and repair, aberrant feedback mechanisms between cells and their remodelled ECM trigger a vicious cycle that leads to disease progression.

In the lung, ECM is typically restricted to basement membranes and the interstitial spaces that form the parenchyma. Within the lung interstitium, resident fibroblasts are the most commonly identified cell and are mainly responsible for ECM production; they also serve as effector cells during injury repair. The ECM produced plays an active role in shaping the behaviour of the fibroblast and other cells. Its quality (based on composition, fibre orientation and stiffness) provides critical cues that help orient cells to their location and provide information about their environment. Both the ECM and the fibroblast are therefore critical players in the early and late wound repair responses in the lung.

The compositions and methods disclosed herein support and promote lung epithelial ECM damage repair through production of new collagen and elastic fibre networks. Such ECM repair thereby improves the mechanical properties of the lung, gaseous exchange in the long term, and decreases susceptibility to future infections and disease.

According to a first aspect of the present invention, there is provided a composition for use in the treatment of disease of the airways in a subject, the composition comprising either a first tetrapeptide, a second peptide, a third tetrapeptide, a fourth tetrapeptide or a combination of the first tetrapeptide and the second peptidea wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid selected from the group consisting of Valine (V), Aspartic acid (D), Proline (P), Glycine (G) and mixtures thereof, at the N-terminal end, U is selected from the group consisting of H, -CO-R¹, -SO₂-R¹ or a biotinyl group, at the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid selected from the group consisting of Lysine (K), Glutamic acid (E) and Serine (S) and mixtures thereof, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

According to a second aspect, the present invention provides a method of treating a disease of the airways in a subject, wherein the method comprises administering a composition to the airways of a subject the composition comprising either a first tetrapeptide, a second peptide, a third tetrapeptide, a fourth tetrapeptide or a combination of the first tetrapeptide and the second peptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, or a pentapeptide having the amino acid sequence according to SEQ ID No: 17, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a third aspect there is provided a composition suitable for inhalation, the composition comprising either a first tetrapeptide, a second peptide, a third tetrapeptide, a fourth tetrapeptide or a combination of the first tetrapeptide and the second peptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, or a pentapeptide having the amino acid sequence according to SEQ ID No: 17, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a fourth aspect, the present invention provides a kit comprising an inhaler or a nebuliser and a composition according to the third aspect.

### Detailed description of the invention

The present invention is directed toward compositions for use in the treatment of a disease of an airway in a subject, and methods of treating a disease of the airway in a subject comprising administering a composition to the airways of a subject.

The composition and method enable the production of proteins of the lung epithelial extracellular matrix (ECM), including at least fibrillin, fibronectin, decorin and collagen.

The subject may be a mammal, such as a human, primate, dog, cat, cattle, pig, horse or sheep. In preferred embodiments, the subject is a human.

The airway comprises the respiratory system including the upper and lower respiratory tract. Parts of the respiratory system include the nose, nasal cavities, sinuses, pharynx, the larynx, the trachea, and all parts of the lung including the bronchi, the bronchioles and the alveoli. In some embodiments, the compositions for use and methods of treatment are directed to the treatment of the lower respiratory tract.

The disease of the airways may be a lung disease such as asthma, acute respiratory distress syndrome (ARDS), asbestosis, bronchiectasis, bronchitis, catarrh, chronic obstructive pulmonary disease (COPD), cough, croup, cystic fibrosis, idiopathic pulmonary fibrosis (IPF), obstructive sleep apnoea, pleurisy, pneumonia, pulmonary hypertension, or pulmonary fibrosis. In one embodiment, the lung disease is COPD.

In one embodiment, the lung disease is asthma. Asthma is the result of chronic inflammation of the airway that leads to exaggerated airway narrowing and stiffening. The structure and organisation of the ECM is key in determining this stiffness.

In one embodiment, the lung disease is ARDS. The acute phase of ARDS is characterized by local and systemic inflammatory responses and is often accompanied by extensive ECM remodelling.

In one embodiment, the lung disease is IPF. IPF is a devastating, progressive fibrosing disease with limited treatment options thought to arise from repetitive damage to the lung epithelium promoting stiffening . It is characterized by extensive and disorganized accumulation of abnormal ECM in the lung parenchyma.

The disease of the airways may be a pharynx disease such as tonsillitis, croup, or pharyngitis. The disease of the airways may be an epiglottis disease such as epiglottitis. The disease of the airways may be a larynx disease such as croup or laryngitis. The disease of the airways may be a trachea disease such as tracheomalacia.

### Tetrapeptides

The present invention is directed toward compositions for use in the treatment of a disease of an airway in a subject, and methods of treating a disease of the airway in a subject comprising administering a composition to the airways of a subject. The compositions comprise a first tetrapeptide, a second peptide or a combination thereof.

The first tetrapeptide (a) has generic formulation U-LSXX-Z wherein L is used to denote amino acid Leucine and S is used to denote Serine, as per the internationally recognised single letter code for amino acids. X denotes an amino acid selected from the group consisting of Valine (V), Aspartic acid (D), Proline (P), Glycine (G) and mixtures thereof. At the N-terminal end, U is selected from the group consisting of H, -CO-R¹, -SO₂-R¹ or a biotinyl group. At the C-terminal end, Z is selected from the group consisting of OH, O R¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

The tetrapeptide of the present invention is preferably selected from the group consisting of SEQ ID No: 1, SEQ ID No: 2, SEQ ID No: 3, SEQ ID No: 4, SEQ ID No: 5, SEQ ID No: 6, SEQ ID No: 7, SEQ ID No: 8, SEQ ID No: 9, SEQ ID No: 10, SEQ ID No: 11 and SEQ ID No: 12.

In another embodiment of the present invention the tetrapeptide is preferably selected from the group consisting of SEQ ID No: 1, SEQ ID No: 9 and SEQ ID No: 8.

In another embodiment of the present invention the tetrapeptide is preferably selected from the group consisting of SEQ ID No: 1.

In another embodiment of the present invention the tetrapeptide is preferably selected from the group consisting of SEQ ID No: 9.

In another embodiment of the present invention the tetrapeptide is preferably selected from the group consisting of SEQ ID No: 8.

In a preferred embodiment the tetrapeptide combination of the present invention, tetrapeptide a) is selected from the group consisting of U-LSVD-Z, U-LSVP-Z, U-LSVG-Z, U-LSDV-Z, U-LSDP-Z, U-LSDG-Z, U-LSPV-Z, U-LSPD-Z, U-LSPG-Z, U-LSGV-Z, U-LSGD-Z and U-LSGP-Z. In another embodiment, the tetrapeptides is selected from the group consisting of U-LSVD-Z, U-LSPG-Z and U-LSPD-Z. In another embodiment the tetrapeptide a) is Pal-LSVD-OH. In another embodiment the tetrapeptide a) is Pal-LSPG-OH. In another embodiment the tetrapeptide a) is Pal-LSPD-OH.

The second peptide (b) has generic amino acid sequence U-GPXG-Z wherein G is used to denote amino acid glycine and P denotes the amino acid proline, as per the internationally recognised single letter code for amino acids, X denotes an amino acid selected from the group consisting of Lysine (K), Glutamic acid (E) and Serine (S) and mixtures thereof, at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a preferred embodiment, peptide b) is selected from the group consisting of SEQ ID No: 13, SEQ ID No: 14, SEQ ID No: 15, SEQ ID No: 16 and SEQ ID No: 17.

In a further preferred embodiment of the present invention peptide b) is U-GPKG-Z.

In a further preferred embodiment of the present invention peptide b) is U-GPEG-Z.

In a further preferred embodiment of the present invention peptide b) is U-GPSG-Z.

The third tetrapeptide (c) has the generic formulation U-XXGD-Z wherein G is used to denote amino acid Glycine and D is used to denote amino acid Aspartic acid, as per the internationally recognised single letter code for amino acids. X denotes an amino acid selected from the group consisting of Glutamic acid (E), Lysine (K), Leucine (L), Alanine (A), Isoleucine (I), Arginine (R) and mixtures thereof. At the N-terminal end, U is selected from the group consisting of H, -CO-R¹, -SO₂-R¹ or a biotinyl group. At the C-terminal end, Z is selected from the group consisting of OH, OR¹, NHR¹ or NR¹R². R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

The third tetrapeptide is preferably selected from the group consisting of SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, SEQ ID No: 41, SEQ ID No: 42, SEQ ID No: 43, SEQ ID No: 44, SEQ ID No: 45, SEQ ID No: 46, SEQ ID No: 47, SEQ ID No: 48, SEQ ID No: 49, SEQ ID No: 50, SEQ ID No: 51, SEQ ID No: 52, SEQ ID No: 53 and SEQ ID No: 54.

In another embodiment the third tetrapeptide is preferably selected from the group consisting of SEQ ID No: 25, SEQ ID No: 36, SEQ ID No: 44 and SEQ ID No: 51.

In another embodiment the third tetrapeptide is preferably selected from the group consisting of SEQ ID No: 25.

In another embodiment the third tetrapeptide is preferably selected from the group consisting of SEQ ID No: 36.

In another embodiment the third tetrapeptide is preferably selected from the group consisting of SEQ ID No: 44.

In another embodiment the third tetrapeptide is preferably selected from the group consisting of SEQ ID No: 51.

In a preferred embodiment the third tetrapeptide is selected from the group consisting of U-EKGD-Z, U-ELGD-Z, U-EAGD-Z, U-EIGD-Z, U-ERGD-Z, U-KEGD-Z, U-KLGD-Z, U-KAGD-Z, U-KIGD-Z, U-KRGD-Z, U-LEGD-Z, U-LKGD-Z, U-LAGD-Z, U-LIGD-Z, U-LRGD-Z, U-IEGD-Z, U-IKGD-Z, U-ILGD-Z, U-IAGD-Z, U-IRGD-Z, U-REGD-Z, U-RKGD-Z, U-RLGD-Z, U-RAGD-Z, U-RIGD-Z, U-AEGD-Z, U-AKGD-Z, U-ALGD-Z, U-AIGD-Z and U-ARGD-Z. In another embodiment, the third tetrapeptides is selected from the group consisting of U-EKGD-Z, U-LKGD-Z, U-IRGD-Z and U-AKGD-Z. In another embodiment the third tetrapeptide is U-EKGD-Z. In another embodiment the third tetrapeptide is U-LKGD-Z. In another embodiment the third tetrapeptide is U-IRGD-Z. In another embodiment the third tetrapeptide is U-AKGD-Z.

The fourth tetrapeptide (d) has the amino acid sequence U-QTAV-Z wherein Q is used to denote amino acid Glutamine, T is used to denote amino acid Threonine, A is used to denote amino acid Alanine and V is used to denote amino acid Valine. At the N-terminal end, U is selected from the group consisting of H, - CO-R¹, -SO₂-R¹ or a biotinyl group. At the C-terminal end, Z is selected from the group consisting of OH, OR¹, NHR¹ or NR¹R². R¹ and R²are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N

Where, at the N-terminal, U is H then the amino acid is not modified. When, at the C-terminal, Z is OH then the amino acid is not modified. The tetrapeptide is thus not in derivatised form. When other than U is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through vectoring, for example by encapsulation of the peptide.

In a preferred embodiment of the present invention the tetrapeptide is modified at the N-terminal and/or the C-terminal end.

In a preferred embodiment of the present invention, R¹ and/or R² is an alkyl chain of from 1 to 24 carbon atoms, preferably a lipophilic alkyl chain of 3 to 24 carbon atoms.

In a further preferred embodiment of the present invention U is an acyl group -CO-R¹ and Z is selected from the group consisting of OH, methoxy, ethoxy and NH₂, preferably OH. In a further embodiment, U is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment of the present U is independently selected from lauroyl (C12), myristoyl (C14) and palmitoyl (C16).

In a further preferred embodiment Z is OH and U is independently selected from the group consisting of palmitoyl (C16), myristoyl (C14) and lauroyl (C12). Most preferably U is palmitoyl (C16) and Z is OH.

The tetrapeptides may comprise amino acids in the D- or L- configuration. The tetrapeptides may comprise an acid C-terminus such as -CO₂H.

The amino acids making up the tetrapeptides according to the invention may be optically pure, be made up of L or D isomers or a mixture thereof. L isomers are those present in the natural state and may be preferred.

The present invention also envisages and includes further derivatives of the tetrapeptide, including for example modification and/or addition of a chemically functional group to one or more of the amino acids but without a change in the carbon skeleton. The present invention also envisages and includes further analogues of the tetrapeptide, including modification and/or addition of a chemically functional group to one or more of the amino acids with a change in the carbon skeletal and complexes of the tetrapeptide with other species such as a metal ion (e.g. copper, zinc, manganese, magnesium, and others).

Tetrapeptides are also envisaged in the form of salts, including hydrochloric salt, or acetate.

The combination of tetrapeptides of the present invention show synergistic benefit in that they offer markedly improved levels of fibrillin-1 production in human dermal fibroblast cells in comparison to the peptides singularly.

### Composition

The present invention also encompasses compositions comprising the tetrapeptides of the present invention. The tetrapeptides are preferably incorporated into the composition in amount of from 0.10ppm to 10,000ppm, preferably from 0.50ppm to 5,000ppm, more preferably from 1ppm to 1000ppm, and most preferably from 1ppm to 500ppm. These are again based on a % w/w basis. Thus 100,000ppm is 10% by weight of the composition.

Where the composition comprises a combination of a first tetrapeptide and a second peptide, the first tetrapeptide and second peptide may be present in the composition in a weight ratio of from 20:80 to 80:20, such as 30:70 to 80:20, 40:60 to 80:20, 50:50 to 80:20, 60:40 to 80:20, or 70:30 to 80:20, based on the total weight of the first tetrapeptide and a second peptide. The first tetrapeptide and second peptide may be present in the composition in a weight ratio of from 20:80 to 80:20, such as 20:80 to 70:30, 20:80 to 60:40, 20:80 to 50:50, 20:80 to 40:60, or 20:80 to 30:70, based on the total weight of the first tetrapeptide and a second peptide. The first tetrapeptide and second peptide may be present in the composition in a weight ratio of from 20:80 to 80:20, such as 30:70 to 70:30, or 40:60 to 60:40, based on the total weight of the first tetrapeptide and a second peptide.

In some embodiments the composition is a pharmaceutical composition. A pharmaceutical composition is a product designed for use by a subject and is preferably a pharmaceutical composition for inhalation into the airways and lungs, where healing is desired.

The composition may comprise common therapeutic agents used in the treatment of respiratory diseases. As such, the composition may comprise the first tetrapeptide and/or second peptide, and one or more common therapeutic agents.

For example, the composition may comprise two or more common therapeutic agents, such as from 2 to 10, 2 to 5, 1 to 4, or 2 to 3 common therapeutic agents.

The common therapeutic agent may be inhaled corticosteroids for the control of inflammation. Examples of inhaled corticosteroids include beclomethasone (Qvar RediHaler^{®}), budesonide (Pulmicort^{®} Flexhaler), ciclesonide (Alvesco^{®}), fluticasone (Flovent HFA^{®}), mometasone (Asmanex Twisthaler^{®}), and combinations thereof.

The common therapeutic agent may be inhaled long-acting beta-agonists to relax the smooth muscles around the airways. Examples of inhaled long-acting beta-agonists include formoterol, indacaterol, salmeterol, vilanterol, and combinations thereof.

The common therapeutic agent may be combination inhaled medicines, such as inhaled corticosteroids in combination with a long-acting beta-agonist. The combination inhaled medicine may additionally comprise an anticholinergic such as aclidinium (Tudorza Pressair^{®}), glycopyrronium (Seebri Neohaler^{®}), ipratropium bromide, tiotropium (Spiriva^{®}), umeclidinium (Incruse^{®} Ellipta), revefenacin (Yupelri^{®}), and combinations thereof. Examples of combination inhaled medicine includes that sold under the brand name Advair^{®} (combination of fluticasone and salmeterol), Breo^{®} (combination of fluticasone and vilanterol), Dulera^{®} (combination of mometasone and formoterol), Symbicort^{®} (combination of budesonide and formoterol), Trelegy^{®} Ellipta (combination of fluticasone, vilanterol, and umeclidinium bromide), and combinations thereof.

The common therapeutic agent may be a cromolyn for the control of inflammation.

The common therapeutic agent may be long-acting bronchodilators. The bronchodilator may be used in combination with inhaled corticosteroids. Examples of long-acting bronchodilators include ipratropium, tiotropium (Spiriva^{®}), theophylline, and combinations thereof.

The composition of the present invention may be aqueous or non-aqueous and comprise of a single-phase system or multiple phase system. Single-phase systems include aqueous and non-aqueous compositions. Multiphase systems include but are not limited to microemulsions, emulsions, and products with discrete separate phases. Emulsions include water-in-oil, oil-in-water emulsions and multiple emulsions (water in oil in water or oil in water in oil for example). The skilled person would understand that the composition must be suitable for inhalation, in that it can be vaporised, or nebulised, aerosolised or dispersed onto a dry powder. The skilled person would be capable of formulating the composition such that it is suitable for inhalation. Examples of methods of preparing a composition suitable for inhalation are included below.

Administration of the composition may be by inhalation orally or intranasally. Administration is preferably via oral inhalation. The components of the composition are preferably adapted to be administered from a dry powder inhaler, a pressurized metered-dose inhaler, or a nebulizer.

Examples of inhalation compositions comprising the composition of the present invention include aerosol formulations, dry powder formulations and nebulized formulations.

### Aerosol compositions

An "aerosol formulation" refers to a formulation that is dispersed into the air using pressurized propellant in the aerosol chamber. One of the most common devices which uses aerosol-based formulations are pressurised metered-dose inhalers (pMDIs). The instrument is able to deliver specific doses of the active ingredient in a propellant or mixture of propellants. The propellant is often liquified, and upon expansion to gaseous form, mobilises the active ingredient.

The composition may be provided in an aerosol formulation. The composition may be in small particle form. The peptide combination may be dissolved or suspended in a liquid propellant mixture. Suitable propellants include hydrofluoroalkanes (HFA) such as HFA134a (1,1,1,2-tetrafluoroethane), HFA 227 (1,1,1,2,3,3,3-heptafluoropropane, HFA152a (1,1-Difluoroethane) and mixtures thereof, and hydrofluoroolefins (HFOs) such as 1,3,3,3-tetrafluoropropene (HFO-1234ze) and 2,3,3,3-tetrafluoropropene (HFO-1234yf), and mixtures thereof. The aerosol formulation may comprise one or more surfactants, such as oleic acid, sorbitan trioleate, or soya lecithin and/or one or more bulking agents, such as lactose. The aerosol formulation may comprise one or more co-solvents known to those skilled in the art, for example alcohols such as ethanol (up to 20% by weight). The aerosol formulation may comprise particles with an average particle size of 10 microns or less, as measured using dynamic light scattering.

### Dry powder compositions

A "dry powder formulation" refers to a formulation that is dispersed into the air in solid particulate form. A dry powder inhaler is an instrument that is capable of generating very fine solid particles for inhalation into the lung. The instrument is a breath activated device, which upon inhalation the active formulation and a carrier are aerosolized from the device and into the airways. Such an instrument may be referred to as a dry powder inhaler.

The composition may be in the form of dry powder formulation, such as a small particle dry powder, or an agglomerated small particle dry powder. The dry powder formulation may further comprise a diluent or carrier, such as lactose, sorbitol, sucrose or mannitol in amounts which facilitate dispersal of the powder from the device. For example, the bulking agent may be present in an amount of from 20 to 90 wt% of the dry formulation. The dry powder may further comprise a compound that helps to protect against product performance deterioration due to moisture, e.g., magnesium stearate. The dry powder formulation may comprise dry powder particles with an average particle size of 10 microns or less, as measured using dynamic light scattering.

### Nebulized compositions

A "nebulized formulation" refers to a solution that is dispersed in the air to form an aerosol. Thus, a nebulized formulation is a particular form of an aerosol. A nebulizer is an instrument that is capable of generating very fine liquid droplets for inhalation into the lung. Within this instrument, the nebulizing liquid or solution is atomized into a mist of droplets with a broad size distribution by methods known to those of skill in the art, including, but not limited to, compressed air, ultrasonic waves, or a vibrating orifice. Soft mist inhalers are a specific type of nebuliser where the mist is produced by high pressure generated during device actuation, such as by releasing a coiled spring. These nebulizers do not need the aid of any external power source. Thus, the mist inhaled into the lung contains fine aerosol droplets. Nebulizers for use herein include, but are not limited, to soft mist inhalers.

The composition comprising the first tetrapeptide, second peptide of combination thereof may be provided in a nebulized formulation. The nebulized formulation may comprise the peptide combination dissolved, or suspended, in a vehicle containing water, a co-solvent, such as ethanol or propylene glycol and a stabilizer, which may be a surfactant. The nebulized formulation may also comprise a buffer and/or a simple sugar, to enable stabilization of the peptides. The nebulized formulation may comprise particles with an average particle size of 10 microns or less, as measured using dynamic light scattering.

### Further peptides

The compositions may comprise further peptides. Preferably said additional peptides are selected from the group consisting of dipeptides, tripeptides, additional tetrapeptides, pentapeptides and mixtures thereof. By tripeptides, it is meant compound comprising an uninterrupted sequence of three amino acids. By tetrapeptides, it is meant a compound comprising an uninterrupted sequence of four amino acids and when using further tetrapeptides, the tetrapeptides are referred to as 'additional tetrapeptides'. By pentapeptide it is meant a compound comprising an uninterrupted sequence of five amino acids.

### Dipeptides:

The compositions may comprise a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide 17, palmitoyl dipeptide 5 diaminobutyroyl hydroxythreonine, palmitoyl dipeptide 5 diaminohydroxybutyrate, palmitoyl dipeptide 7 and mixtures thereof.

Dipeptides are preferably incorporated into the composition of the present invention at a level of from 0.1 to 50000ppm, more preferably from 1 to 5000 ppm, most preferably from 10 to 500ppm.

### Tripeptides:

The emulsions preferably comprise a tripeptide. Said tripeptide may be naturally occurring or of synthetic origin. Suitable tripeptides include tripeptide 1, 2, 3, 4, 5, 6, 7, 8, 9, 10,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However, another suitable tripeptide may be Arg-Lys-Arg. Particularly preferred tripeptides are based on the structure GlU-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., GlU-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the present invention, (and therefore also the more generic term tripeptides). Derivatives of GHK-tripeptides in accordance with the present invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

His-based tripeptides include at least one histidine amino acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline.

Derivatives are also considered to be encompassed by the term His-based tripeptides in accordance with the present invention, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides in accordance with the present invention include N-Acyl-GlU-His-Lys and most preferably, N-Palmitoyl-GlU-His-Lys. Preferred commercially available tripeptide and tripeptide derivative comprising compositions include Biopeptide-CL from SEDERMA, Maxilip(R) from SERMA, Biobustyl(R) from SEDERMA.

The tripeptides where included are preferably incorporated into the composition in amounts of from 0.10ppm to 10,000ppm, preferably from 0.50ppm to 5,000ppm, more preferably from 1ppm to 1000ppm, and most preferably from 1ppm to 500ppm. These are again based on a % w/w basis. Thus 100,000ppm is 10% by weight of the emulsion.

### Additional tetrapeptides:

The composition may comprise an additional tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the present composition include those selected from the group consisting of well-known tetrapeptide 1, 2, 3, 4, 5, 6, 7, 8, 9, 10, 11, 12,13, 14, 15, 16, 17, 18, 19 ,20, 21, 22, 23, 24, 25, 26, 27, 28, 29, 30, 34, 35, derivatives thereof and mixtures thereof.

Rigin-based tetrapeptides in accordance with the present invention are based on the structure Glu-Gln-Pro-Arg (Rigin) and include its analogs and derivatives thereof. Rigin is an additional tetrapeptide. Analogs of the tetrapeptide rigin useful in accordance with the present invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc, Xaa-Xbb-Xcc-Pro, Xaa-Xbb-Pro-Arg, wherein Xaa-Xbb-Pro-Xcc, Xaa-Xbb-Xcc-Arg, Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

Derivatives are also considered to include peptide-divalent ion complexes. Cu2+-peptide derivatives are preferred as this may provide increased biological effect compared to the peptide alone.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH2 comprising side chain. These amino acids include a side chain that has an amine group that is predominantly uncharged at neutral pH (pH 6-7) such as, without limitation, Gln, Asn, Lys, Orn, 5-hydroxyproline, Citrulline and Canavanine. The ALAMCAT-tetrapeptides also include at least one amino acid having at least one side chain including at least one cationic amine (predominant species is charged such as NH3+ , NH2+, etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an aliphatic group, pendant amino group or pendant cationic group. Derivatives are also considered to be encompassed by the term ALAMCAT-tetrapeptides in accordance with the present invention, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys, N-acyl-GlU-Gln-Pro-Arg peptides, most preferably N-palmitoyl-GlU-Gln-Pro-Arg.

Preferred commercially available sources of tetrapeptides include RIGIN, EYELISS, Haloxyl, and MATRIXYL 3000, which comprise between 50 to 500 ppm of palmitoyl-GlU-Gln-Pro-Arg, and other ingredients, such as peptides, chalcones and an excipient, commercially available from SEDERMA, France. Tego Pep 417 available from Evonik. These may be used to produce compositions of the present invention by adding thereto at least one tripeptide as described herein.

The additional tetrapeptides when used are preferably incorporated into the composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

The combination of tripeptides and additional tetrapeptides, can be particularly preferred. When present, the preferred ratio of additional tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of additional tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of additional tetrapeptide used when considered in terms of their amounts in parts per million, again based on overall weight of the composition. In a particularly preferred embodiment, the composition of the present invention comprises an additional tetrapeptide of the sequence GlU-Gln-Pro-Arg, its analogs and derivatives in combination with one or more tripeptide of the sequences GlU-His-Lys, its analogs and derivatives.

### Pentapeptides:

The compositions of the present invention may optionally comprise a pentapeptide, derivatives of pentapeptides, and mixtures thereof. As used herein, "pentapeptides" refers to both the naturally occurring pentapeptides and synthesized pentapeptides. Also, useful herein are naturally occurring and commercially available compositions that comprise pentapeptides. Suitable pentapeptides are those selected from the group consisting of pentapeptide 1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof.

Suitable pentapeptides for use herein are the pentapeptide, lys-thr-thr-lys-ser, Arg-asp-lys-tyr-val (pentapeptide -1) and derivatives thereof. A preferred commercially available pentapeptide derivative-comprising composition is Matrixyl which comprises 100 ppm of palmitoyl-lys-thr-thr-lys-ser and is commercially available from Sederma, France.

The pentapeptides when used are preferably incorporated into the composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

### Hexapeptides:

The compositions may optionally comprise a hexapeptide, derivatives of hexapeptides, and mixtures thereof. As used herein, "hexapeptides" refers to both the naturally occurring hexapeptides and synthesized hexapeptides. Also, useful herein are naturally occurring and commercially available compositions that comprise hexapeptides.

The hexapeptides when used are preferably incorporated into the pharmaceutical composition in amounts from 0.1 ppm (0.00001% w/w also referred to herein as "weight percent", "weight %" or simply by weight) to 10,000 ppm (1.0% w/w), preferably from 0.5 ppm to 1000 ppm (0.1% w/w), and most preferably from 1 ppm to 500ppm (0.05% w/w) by weight of the composition.

### Antioxidant agents

The compositions may optionally comprise an antioxidant agent. Suitable antioxidant agents may include: a) ascorbic acid its salts, esters, glucosides and glucosamines, particularly sodium ascorbyl phosphate, magnesium ascorbyl phosphate, ascorbyl palmitate and ethyl ascorbic acid b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts, particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, panax ginseng, such as that available under the trade name "Panax ginseng 1.1 extract 4294"from S Black Ltd or "Phytexcell Panax ginseng" available from Croda Chemicals Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica^{™} by Merck Speciality chemicals, and Grape Seed oil, such as that available from Chesham Chemicals Limited.

The amounts of antioxidant agents used in the pharmaceutical composition are expressed as dry weights, as understood by a man skilled in the art. The total amount of antioxidant agents optionally present in the composition may range from 0.005% to 10% by weight, preferably 0.5% to 5%, most preferably 0.2% to 1.5% by weight of the composition.

Particularly preferred synergistic combinations of antioxidant agents suitable for inclusion in the pharmaceutical composition are selected from the group consisting of : i) panax ginseng, morus alba and magnesium ascorbyl phosphate; ii) panax ginseng, morus alba and sodium ascorbyl phosphate; iii) panax ginseng, morus alba and rosmarinus officinalis; iv) ginkgo biloba, phyllanthus emblica and Dimethylmethoxy chromanol; v) morus alba, camellia sinensis and dimethylmethoxy chromanol; vi) morus alba, camellia sinensis and tocopheryl acetate; vii) panax ginseng, morus alba and origanum vulgare, viii) camellia sinensis, tocopheryl acetate and dimethylmethoxychromanol, viv) morus alba, tocopheryl acatete and dimethylmethoxychromanol.

In these preferred combinations (a) the panax ginseng is preferably present in an amount of 0.005% to 0.1%, more preferably 0.01% to 0.05% by weight of the composition; (b) the morus alba is preferably present in an amount of 0.0005% to 0.01%, more preferably 0.001% to 0.005% by weight of the composition; (c) the sodium, magnesium ascorbyl phosphate or ethyl ascorbic acid is preferably present in an amount of 0.05% to 2.5%, preferably 0.1% to 2%, most preferably 0.15% to 1.5% by weight of the composition;(d) the rosmarinus officinalis or origanum vulgare or phyllanthus emblica is preferably present in an amount of 0.01% to 0.5%, more preferably 0.05% to 0.2% by weight of the composition e) the dimethylmethoxy chromanol is preferably present in an amount of 0.0005% to 0.1%, more preferably from 0.005% to 0.05% by weight of the composition ; f) the camellia sinensis is preferably present in an amount of 0.005% to 0.2%, more preferably from 0.01% to 0.1% and the g) Tocopherol acetate is preferably present in an amount of 0.01 to 0.5%, more preferably from 0.05% to 0.25%

### Vitamins

The compositions may comprise one or more vitamins. The compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid. The composition may comprise vitamin B, vitamin B derivatives, vitamin B1 to vitamin B12 and their derivatives. In a further embodiment the composition comprising the Vitamin B3 derivative niacinamide.

In an alternative embodiment of the present invention the pharmaceutical composition comprises vitamin K, vitamin K derivatives, vitamin H, vitamin D, vitamin D derivatives and mixtures thereof. In an alternative embodiment of the present invention the pharmaceutical composition comprises vitamin E, vitamin E derivatives such as tocopherol and tocopheryl acetate, and provitamins thereof, such as panthenol and mixtures thereof.

In a further embodiment the present pharmaceutical composition comprises retinoid compounds, including retinoic acid, retinaldehyde, retinol and derivatives thereof. In one embodiment the pharmaceutical composition comprises retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e. g. plant) sources. In one embodiment, when vitamin compounds are present in the compositions of the instant invention, the emulsion compositions comprise from about 0.0001% to 50%, more preferably from 0.001% to 10%, still more preferably from 0.01% to 8%, and still more preferably from 0.1% to 5%, by weight of the composition, of the vitamin compound.

### Methods of use

The present invention also relates to a method of treating a disease of the airways in a subject. The method comprises administering to said subject the composition (e.g. in a therapeutically effective amount) as defined herein.

The method may comprise administration of the composition by inhalation orally or intranasally. Administration is preferably via oral inhalation. The components of the composition are preferably adapted to be administered from a dry powder inhaler, a pressurized metered-dose inhaler, or a nebulizer.

### Composition suitable for inhalation

The present invention also related to compositions suitable for inhalation. The composition may be made suitable for inhalation according to methods and techniques known in the art.

For example, in some embodiments the composition may be provided in an aerosol formulation. The composition may be in small particle form. The peptide combination may be dissolved or suspended in a liquid propellant mixture. Suitable propellants include hydrofluoroalkanes (HFA) such as HFA134a (1,1,1,2-tetrafluoroethane), HFA 227 (1,1,1,2,3,3,3-heptafluoropropane, HFA152a (1,1-Difluoroethane) and mixtures thereof, and hydrofluoroolefins (HFOs) such as 1,3,3,3-tetrafluoropropene (HFO-1234ze) and 2,3,3,3-tetrafluoropropene (HFO-1234yf), and mixtures thereof. The aerosol formulation may comprise one or more surfactants, such as oleic acid, sorbitan trioleate, or soya lecithin and/or one or more bulking agents, such as lactose. The aerosol formulation may comprise one or more co-solvents known to those skilled in the art, for example alcohols such as ethanol (up to 20% by weight).

In some embodiments, the composition may be in the form of dry powder formulation, such as a small particle dry powder, or an agglomerated small particle dry powder. The dry powder formulation may further comprise a diluent or carrier, such as lactose, sorbitol, sucrose or mannitol in amounts which facilitate dispersal of the powder from the device. For example, the bulking agent may be present in an amount of from 20 to 90 wt% of the dry formulation. The dry powder may further comprise a compound that helps to protect against product performance deterioration due to moisture, e.g., magnesium stearate. The dry powder formulation may comprise dry powder particles with an average particle size of 10 microns or less, as measured using dynamic light scattering.

In some embodiments, the composition may be provided in a nebulized formulation. The nebulized formulation may comprise the peptide combination dissolved, or suspended, in a vehicle containing water, a co-solvent, such as ethanol or propylene glycol and a stabilizer, which may be a surfactant. The nebulized formulation may also comprise a buffer and/or a simple sugar, to enable stabilization of the peptides.

### Kit

The present invention also relates to a kit comprising the composition suitable for inhalation as described herein and an inhaler or a nebuliser.

### Examples

The present invention is further described by the following examples.

### Examples of tetrapeptide synthesis

The tetrapeptides of the present invention with generic formulation pal-X¹X²X³X⁴-OH are prepared by peptidic synthesis. In a first step the N-terminal of X⁴ is coupled with a resin via the terminal acid functionality in the presence of a coupling agent. The N terminal amine is then reacted with the next amino acid in the sequence X³ in the presence of a coupling agent. The same process is repeated until the required sequence is obtained and a suitable C terminal functionality added. Suitable coupling agents include DCC (dicyclohexylcarbodiimide)/NHS (N-hydroxysuccinimide) or HBTU (2-(1H-benzotriazole-1-yl)-1,1,3,3-tetramethyluronium hexafluorophosphate)/HOBT (1-hydroxy-benzotriazole)). The resulting peptide is then cleaved from the resin in an acidic medium and after precipitation, washing and drying, the peptide is obtained in solid form.

### Example Compositions

### Metred dose inhaler

| **Ingredient** | **Concentration** |
|---|---|
| Budesonide (corticosteroid) | 200ug/inhalation |
| Formoterol fumerate dihydrate (Long-acting beta agonist) | 4.5ug/inhalation |
| Hydrofluoroalkane-134a | 100ug/inhalation |
| Peptide | 100ug/inhalation |
| Lactose monohydrate (excipient) | 700ug/inhalation |

### Method of manufacture

1. Drug ingredients Budesonide and Formoterol are added into a vial containing ethanol (solvent) and mixed to form a solution.
2. Hydrofluoroalkane-134a (HFA) which serves as the propellant is then placed into a pre-chilled vessel.
3. The concentrated drug solution is then transferred into the vessel, lactose monohydrate added and peptide added and the entire formulation is mixed.
4. A predetermined portion of the chilled liquid formulation is then feed into an open canister.
5. A valve is placed on top of each canister and then crimped into place.

### Dry powder inhaler

| **Ingredient** | **Concentration** |
|---|---|
| Salbutamol sulfate | 100ug/inhalation |
| Peptide | 100ug/inhalation |
| Lactose | 10mg/inhalation |

1. Organic phase microparticles are first created through solvent evaporation by preparing an oil/water emulsion with Poly Lactic-co-glycolic dissolved in dichloromethane in a 50:50 ratio.
2. Salbutamol sulfate is then added and the solution mixed by sonication using probe sonicator and injected this mixture drop wise into an aqueous phase containing the peptides and 2% w/v PVA in water.
3. The mixture is homogenized at 10 000 rpm for 10 min using a homogenizer (Viritis Cyclone IQ, USA) to create an emulsion.
4. The emulsion is stirred for 12 h at 25±2° using magnetic stirrer to ensure complete evaporation of dichloromethane.
5. The microparticles thus prepared are recovered by centrifugation (15 000 rpm, 20 min, 4°).
6. The precipitate is washed to remove the polyvinyl alcohol.
7. The product is finally dispersed in cold water and recovered by lyophilisation.

## Claims

1. A composition for use in the treatment of disease of the airway in a subject, the composition comprising either a first tetrapeptide, a second peptide, a third tetrapeptide, a fourth tetrapeptide or a combination of the first tetrapeptide and the second peptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysin(K), Glutamic acid (E), Proline (P) and Serine (S) and mixtures thereof, or a pentapeptide having the amino acid sequence according to SEQ ID No: 17, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R¹, -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

2. A method of treating a disease of the airways in a subject, wherein the method comprises administering a composition to the airways of a subject, the composition comprising either a first tetrapeptide, a second peptide, a third tetrapeptide, a fourth tetrapeptide or a combination of the first tetrapeptide and the second peptide, wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, or a pentapeptide having the amino acid sequence according to SEQ ID No: 17, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R¹, -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

3. The composition for use in claim 1, or the method of claim 2, wherein the subject is a human.

4. The composition for use of any one of claims 1 or 3, or the method of use of any one of claims 2-3, wherein the composition is administered via inhalation.

5. A composition suitable for inhalation, the composition comprising either a first tetrapeptide, a second peptide, a third tetrapeptide, a fourth tetrapeptide or a combination of the first tetrapeptide and the second peptide wherein:
a) the first tetrapeptide is selected from the group consisting of tetrapeptides having the amino acid sequence U-LSXX-Z wherein L is used to denote amino acid Leucine and S is used to denote Serine, as per the internationally recognised single letter code for amino acids, X denotes an amino acid independently selected from the group consisting of Valine (V), Aspartic acid (D), Proline (P), Glycine (G), at the N-terminal end, U is selected from the group consisting of H, -CO-R¹, -SO₂-R¹ or a biotinyl group, at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
b) the second peptide is selected from the group consisting of tetrapeptides having the amino acid sequence U-GPXG-Z wherein G is used to denote amino acid glycine and P denotes the amino acid proline, as per the internationally recognised single letter code for amino acids, X denotes an amino acid independently selected from the group consisting of Lysine(K), Glutamic acid (E), Proline (P) and Serine (S) and mixtures thereof, or a pentapeptide having the amino acid sequence according to SEQ ID No: 17, at the N-terminal end, U is selected from the group consisting of H, -CO-R¹, - SO₂-R¹ or a biotinyl group, at the C-terminal end, Z is selected from the group consisting of OH, O R¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N;
c) the third tetrapeptide is selected from the group consisting of tetrapeptides having the amino acid sequence U-XXGD-Z wherein G is used to denote amino acid Glycine and D is used to denote amino acid Aspartic acid, as per the internationally recognised single letter code for amino acids; X denotes an amino acid selected from the group consisting of Glutamic acid (E), Lysine (K), Leucine (L), Alanine (A), Isoleucine (I), Arginine (R) and mixtures thereof; at the N-terminal end, U is selected from the group consisting of H, -CO-R¹, -SO₂-R¹ or a biotinyl group; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N; and
d) the fourth tetrapeptide is a tetrapeptide having the amino acid sequence U-QTAV-Z wherein Q is used to denote amino acid Glutamine, T is used to denote amino acid Threonine, A is used to denote amino acid Alanine and V is used to denote amino acid Valine; at the N-terminal end, U is independently selected from the group consisting of octanoyl (C8), decanoyl (C10), lauroyl (C12), myristoyl (C14), palmitoyl (C16), stearoyl (C18), biotinoyl, elaidoyl, oleoyle, lipoyle; at the C-terminal end, Z is selected from the group consisting of OH, OR¹, NHR¹ or NR¹R²; R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

6. The composition for use of any one of claims 1 or 3-4, the method of use of any one of claims 2-4, or the composition of claim 4 wherein the tetrapeptide a) is selected from the group consisting of SEQ ID No: 1, SEQ ID No: 2, SEQ ID No: 3, SEQ ID No: 4, SEQ ID No: 5, SEQ ID No: 6, SEQ ID No: 7, SEQ ID No: 8, SEQ ID No: 9, SEQ ID No: 10, SEQ ID No: 11 and SEQ ID No: 12, preferably the group consisting of SEQ ID No: 1, SEQ ID No: 9 and SEQ ID No: 8.

7. The composition for use, the method of use, or the composition of any preceding claim, wherein tetrapeptide a) is U-LSVD-Z, U-LSPG-Z, or U-LSPD-Z.

8. The composition for use, the method of use, or the composition of any preceding claim wherein the peptide b) is selected from the group consisting of SEQ ID No: 13, SEQ ID No: 14, SEQ ID No: 15, SEQ ID No: 16 and SEQ ID No: 17.

9. The composition for use, the method of use, or the composition of any preceding claim, wherein peptide b) is U-GPKG-Z, U-GPEG-Z or U-GPSG-Z.

10. The composition for use, the method of use, or the composition of any preceding claim wherein U of the tetrapeptide is independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle, preferable the group consisting of lauroyl (C12), myristoyl (C14) and palmitoyl (C16).
10. The composition for use, the method of use, or the composition of any preceding claim wherein the third tetrapeptide (c) is selected from the group consisting of SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34, SEQ ID No: 35, SEQ ID No: 36, SEQ ID No: 37, SEQ ID No: 38, SEQ ID No: 39, SEQ ID No: 40, SEQ ID No: 41, SEQ ID No: 42, SEQ ID No: 43, SEQ ID No: 44, SEQ ID No: 45, SEQ ID No: 46, SEQ ID No: 47, SEQ ID No: 48, SEQ ID No: 49, SEQ ID No: 50, SEQ ID No: 51, SEQ ID No: 52, SEQ ID No: 53 and SEQ ID No: 54, preferably from the list consisting of SEQ ID No: 25, SEQ ID No: 36, SEQ ID No: 44 and SEQ ID No: 51.

11. The composition for use, the method of use, or the composition of any preceding claim wherein the third tetrapeptide (c) is selected from the group consisting of Pal-AKGD-OH, Pal-EKGD-OH, Pal-LKGD-OH, Pal-IRGD-OH.

12. The composition for use, the method of use, or the composition of any preceding claim, wherein a peptide is present at from 0.1ppm to 10,000ppm by weight of the composition.

13. The composition for use, the method of use, or the composition of any preceding claim, wherein the composition further comprises additional further peptides selected from the group consisting of dipeptides, tripeptides, additional tetrapeptides, pentapeptides, hexapeptides and mixtures thereof.

14. The composition for use, the method of use, or the composition of any preceding claim, wherein the tetrapeptide a) and peptide b) are present in the composition in a weight ratio of from 20:80 to 80:20 based on the total weight of peptides a) and b).

15. A kit comprising:
i) the composition as defined in any one of claims 5-14; and
ii) an inhaler or nebulizer.
